# EUROPEAN PATENT APPLICATION

(11) **EP 3 666 291 A1**
(43) Date of publication of application: **17.06.2020**
(21) Application number: 18844894.8
(22) Date of filing: 03.08.2018
(51) Int. Cl.: A61L 2/06, A61L 2/26, A61L 9/22, F26B 21/02

(54) **DEVICE FOR STERILIZING/DRYING/DEODORIZING HATS INCLUDING SAFETY HATS**

(30) Priority: 07.08.2017 KR 20170099575
(71) Applicant: Yoon, Sek Hun, Bucheon-si, Gyeonggi-do 14550 (KR)
(72) Inventor: Yoon, Sek Hun, Bucheon-si, Gyeonggi-do 14550 (KR)
(74) Representative: dompatent von Kreisler Selting Werner - Partnerschaft von Patent- und Rechtsanwälten mbB
(86) International application number: PCT/KR2018/008867
(87) International publication number: WO 2019/031777

(57) **Abstract**

The present invention relates to a device for sterilizing/drying/deodorizing hats including safety hats, which can sterilize, deodorize, and dry safety hats worn in industrial sites, motorcycle helmets, and normal hats. In particular, the device for sterilizing/drying/deodorizing hats including safety hats, according to the present invention is characterized by comprising: a case main body having an installation hole portion, an air guide ring portion, an installation ledge portion having multiple fastening holes, a safety hat seating groove portion, a hanger portion, retaining holders, and fastening bosses; a case bottom plate; an intake fan; an air induction portion; a filter member; a fixing frame body; a deodorizing ball containing chest body; deodorizing balls; a filter case; ion generating portions; and a control member.

## Description

### [Technical Field]

The present invention relates to a sterilizing/drying/deodorizing apparatus for hats including safety helmets, which may rapidly and conveniently sterilize, deodorize and dry safety helmets used at an industrial site, motorcycle helmets, general hats, etc.

### [Background Art]

As is well known, helmets are worn by persons riding motorcycles, safety helmets are worn by workers on industrial sites so as to protect the workers' heads, and various types of hats are worn for style.

In particular, an industrial site is exposed o various dangerous factors, such as falling objects, and a worker wears a safety helmet as a basic means for safely protecting the worker's head from these dangerous factors.

Such a safety helmet includes an outer cover which primarily absorbs external shock, and a support which is mounted in the outer cover to surround the worker's head, and the safety helmet is easily wet with sweat and is contaminated to cause bad smells due to a dissatisfactory ventilation structure thereof, and thus when the worker wears the safety helmet again, the safety helmet causes displeasure.

Therefore, it is necessary to periodically wash this safety helmet, but there is no proper means and thus the safety helmet is merely washed using only tap water.

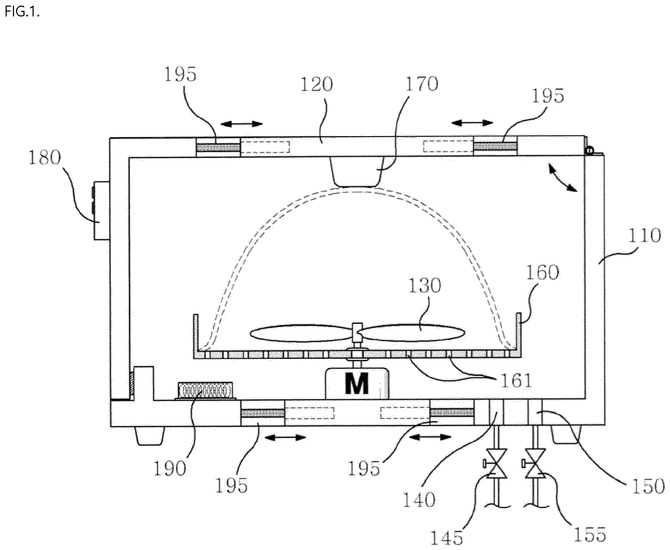

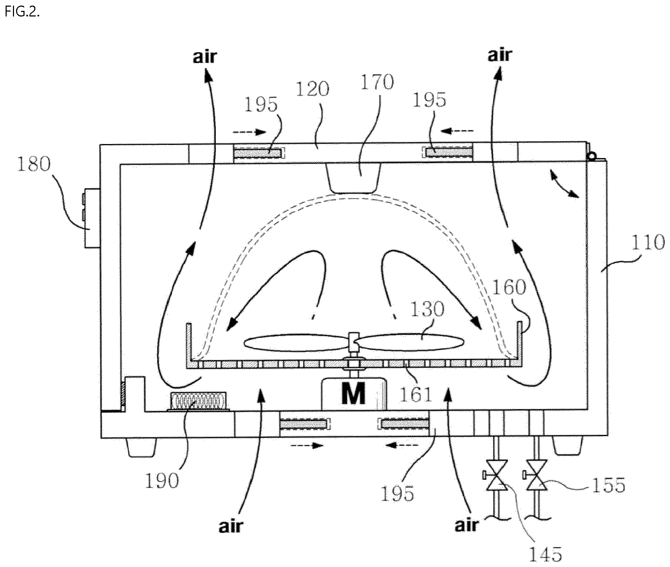

Thus, a safety helmet washer for easily washing safety helmets is disclosed in Korean Patent Unexamined Publication No. 10-2014-0106228, and referring to FIGs. 1 and 2, the safety helmet washer includes a case 110 having a space for accommodating a safety helmet, a cover unit 120 coupled to the case 110 so as to be opened and closed, rotating blades 130 installed on the bottom of the inside of the case 110 and rotated by a motor 135, a washing water inlet 140 formed in the case 110 to supply washing water to the inside of the case 110 therethrough, and a washing water outlet 150 formed in the bottom of the case 110 to discharge the washing water to the outside.

That is, the safety helmet washer having the above configuration may rapidly and conveniently wash the safety helmet at an industrial site. That is to say, washing water is uniformly stirred using the rotating blades 130 operated by the motor 135, thereby being capable of rapidly and conveniently washing the safety helmet.

Further, in addition to washing of the safety helmet, the safety helmet washer may rapidly dry the safety helmet so that a worker may immediately wear the safety helmet. That is, the washed safety helmet is dried using an airflow generated by the rotating blades 130, and, if a smooth airflow is generated by opening ventilating openings 195 and heat of a heat generator 190 is used, a hot airflow is generated and thus a drying time may be further shortened.

Finally, washing and drying are automatically controlled and thus the safety helmet washer may be more conveniently used. That is to say, an automatic controller 180 automatically controls operation of the motor 135, an inlet valve 145, an outlet valve 155, the ventilating openings 195 and the heat generator 190, and when a user merely operates an operation button or touches a touch screen of the automatic controller 180, washing and drying processes are automatically executed.

However, the conventional safety helmet washer having the above configuration washes safety helmets using water, thereby causing serious environmental pollution due to water pollution and consuming a great quantity of water.

Further, the above conventional safety helmet washer which is bulky is not problematic for personal use for helmets or general hats, but if many workers at an industrial site want to wash their safety helmets simultaneously, as many expensive safety helmet washers as the number of the helmets should be provided, thus causing a major expenditure and requiring a large separate installation place on the industrial site due to the large volume of the safety helmet washers.

### (Prior Art Document)

### (Patent Document)

(Patent Document 1) Korean Patent Unexamined Publication No. 10-2014-0106228

### [Disclosure]

### [Technical Problem]

Therefore, the present invention has been made in view of the above problems, and it is an object of the present invention to provide a sterilizing/drying/deodorizing apparatus for hats including safety helmets, which injects room temperature air and heated air to the inside of a hat for a designated time so as to dry the hat, and simultaneously, again suctions contaminated air exhausted to the inside of the hat through a filter and then injects purified air to the inside of the hat so as to remove the smell of sweat and bacteria, and thereby does not require water to wash the hat and thus prevents pollution and waste of water for washing the contaminated hat, prevents spread of unpleasant smells to the outside of the hat and thus maintains comfort in a surrounding environment, has a compact size, and is mountable on the wall in a working place and thus does not require a separate installation space for washing contaminated hats.

Further, it is another object of the present invention to provide a plurality of sterilizing/drying/deodorizing apparatuses for hats including safety helmets, which is connected so as to be be conveniently installed, and simultaneously, has a simple wire arrangement so as to supply power therebetween.

### [Technical Solution]

In accordance with the present invention, the above and other objects can be accomplished by the provision of a sterilizing/drying/deodorizing apparatus for hats including safety helmets, the sterilizing/drying/deodorizing apparatus including a case main body configured to have a cylindrical installation hole part formed at a center of an upper portion thereof so as to extend vertically, an air guide ring part formed at a predetermined interval from an inner circumferential surface of the installation hole part so that an air vent is provided between the installation hole part and the air guide ring part, a safety net formed on an upper portion of an inside of the air guide ring part, a installation protrusion part configured to have a plurality of fastening holes formed in the upper portion of the inside of the air guide ring part, a safety helmet seated recess part formed in an upper portion of the case main body close to the installation hole part so that a safety band protruding from a hat, including a safety helmet, is stably mounted on the safety helmet seated recess part, a hanger part installed at a center of the safety helmet seated recess part so that the safety helmet is stably hung on the hanger part, fixing holders installed at both sides of a lower portion of the installation hole part so that a front surface portion of the safety helmet is stably and fixedly inserted into the fixing holders, and a plurality of fastening bosses formed on an open bottom surface of the case main body; a case bottom plate coupled to the open bottom surface of the case main body by a plurality of fastening bosses and screws; an intake fan fixedly coupled to a bottom portion of the installation hole part of the case main body close to the safety net; an air induction part connected to an end of the installation hole part of the case main body and configured to have an air distribution protrusion configured to have a conical shape and to protrude upwards from a central portion of the air induction part to suction external air by the intake fan without colliding therewith and to raise the air along an inner circumferential surface of the air induction part so as to exhaust the air to the air vent between the installation hole part and the air guide ring part; a cylindrical filter case configured to have an installation net formed on an upper surface of the filter case, a disc-shaped filter member installed under a bottom surface of the installation net, a net-shaped fixing frame body coupled to a bottom surface of the filter member so as to stably fix the filter member from below, a deodorizing ball accommodation cabinet including a plurality of intake holes formed in a central portion of a bottom surface of the fixing frame body, and deodorizing balls configured to completely fill an inside of the deodorizing ball accommodation cabinet through an open upper surface thereof so as to remove unpleasant smells from air primarily passed through the filter member, wherein the open upper surface of the deodorizing ball accommodation cabinet is closed by the filter member, and fastening parts corresponding to the fastening holes of the installation protrusion part on the upper portion of the air guide ring part are formed on the bottom surface of the fixing frame body; ion generators respectively installed on both sides of the air induction part to remove smells and various bacteria from inflowing air; and a control member configured to have a plurality of operation buttons so as to control operation of the intake fan and the ion generators installed within the case main body for a designated time.

Further, a sensor unit may be installed on an upper surface of the case main body so as to operate the control member for a designated time when the safety helmet is mounted on the case main body.

Two case main bodies arranged close to each other may be stably fixed to each other by forming connection grooves configured to be opened sideways to a designated depth in both side portions of each of case bottom plates of the case main bodies arranged close to each other, and inserting one H-shaped connection holder into neighboring connection grooves of the case main bodies, which are close to each other.

In addition, female terminal parts configured to receive applied power may be respectively formed at both sides of each of case bottom plates of case main bodies, an external power supply may be connected to one female terminal part of the case bottom plate, located at an outermost position, and power may be supplied to other case bottom plates disposed close to the case bottom plate connected to the external power supply using a jump wire having male terminal parts provided at both ends thereof.

### [Advantageous effects]

The sterilizing/drying/deodorizing apparatus having the above configuration according to the present invention stably fixes a safety helmet to the upper portion of the case main body, suctions air into the central portion of the case main body from the inside of the safety helmet using the intake fan and simultaneously filters the suctioned contaminated air using the filter member, the deodorizing balls and the ion generators, and then exhausts purified air to the inner circumferential surface of the safety helmet through the air vent provided between the installation hole part of the case main body and the air guide ring part, thereby sterilizing, drying and deodorizing the safety helmet without washing using water as in the conventional apparatus.

Further, the sterilizing/drying/deodorizing apparatus according to the present invention simultaneously performs air intake and exhaust through the upper portion of the case main body on which the safety helmet is mounted, such that fresh air is exhausted to the inside from the edge of the safety helmet and simultaneously air is suctioned to the inside of the case main body from the central portion of the safety helmet, thereby preventing unpleasant smells generated from the safety helmet from contaminating an indoor space.

In addition, if there are many workers, multiple case main bodies are conveniently and fixedly connected by inserting each of connection holders into corresponding connection grooves of the case main bodies being in surface contact with each other, an external power supply is connected to one case main body located at the outermost position, among the multiple case main bodies, and power is supplied to other main case bodies disposed close to the main case body connected to the external power supply using a jump wire, thereby allowing multiple sterilizing/drying/deodorizing apparatuses to be easily installed.

### [Description of Drawings]

FIG. 1 is a longitudinal-sectional view of a conventional safety helmet washer,
FIG. 2 is a longitudinal-sectional view illustrating the usage state of the safety helmet washer of FIG. 1,
FIG. 3 is an exploded perspective view of a sterilizing/drying/deodorizing apparatus according to the present invention,
FIG. 4 is an exploded perspective view of the sterilizing/drying/deodorizing apparatus according to the present invention, as seen from below,
FIG. 5 is an exploded perspective view of a principal part extracted from the sterilizing/drying/deodorizing apparatus according to the present invention,
FIG. 6 is a perspective view of the sterilizing/drying/deodorizing apparatus according to the present invention,
FIG. 7 is a perspective view illustrating the usage state of the sterilizing/drying/deodorizing apparatus according to the present invention,
FIG. 8 is a perspective view illustrating the connected state of the sterilizing/drying/deodorizing apparatuses according to the present invention,
FIG. 9 is a perspective bottom view illustrating the connected state of the sterilizing/drying/deodorizing apparatuses according to the present invention,
FIG. 10 is a longitudinal-sectional view of the sterilizing/drying/deodorizing apparatus according to the present invention, and
FIG. 11 is a longitudinal-sectional view illustrating the usage state of the sterilizing/drying/deodorizing apparatus according to the present invention.

### [Best Mode]

Hereinafter, exemplary embodiments of the present invention will be described in detail with reference to the accompanying drawings.

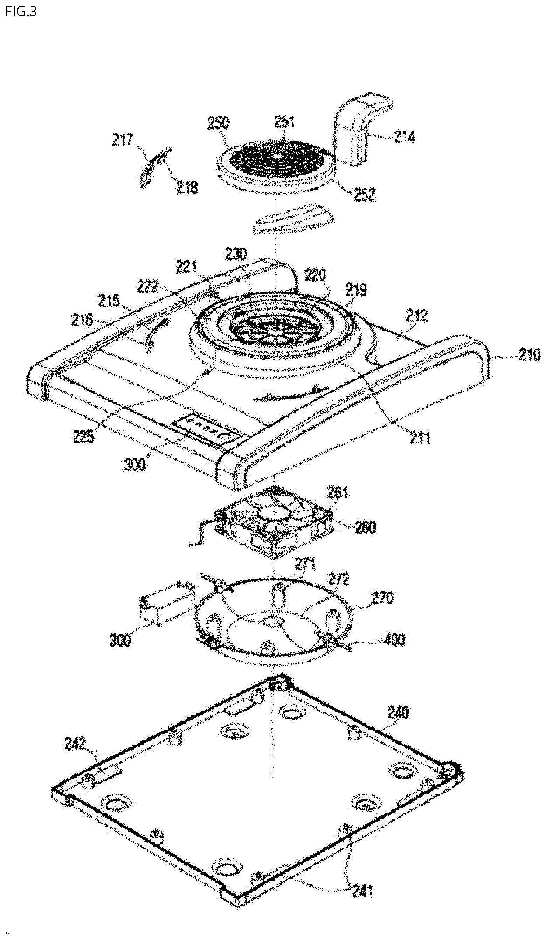

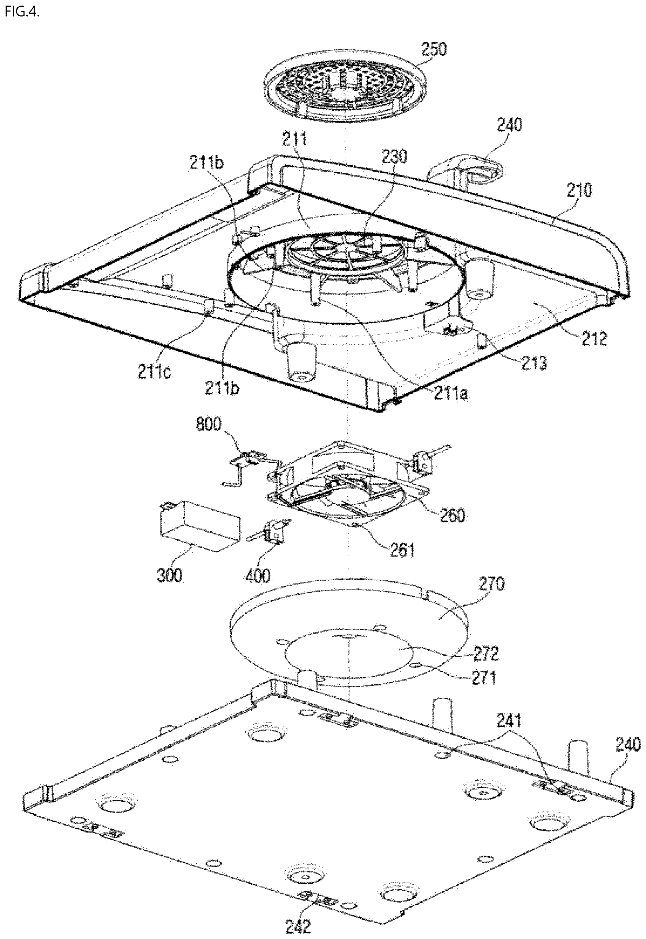

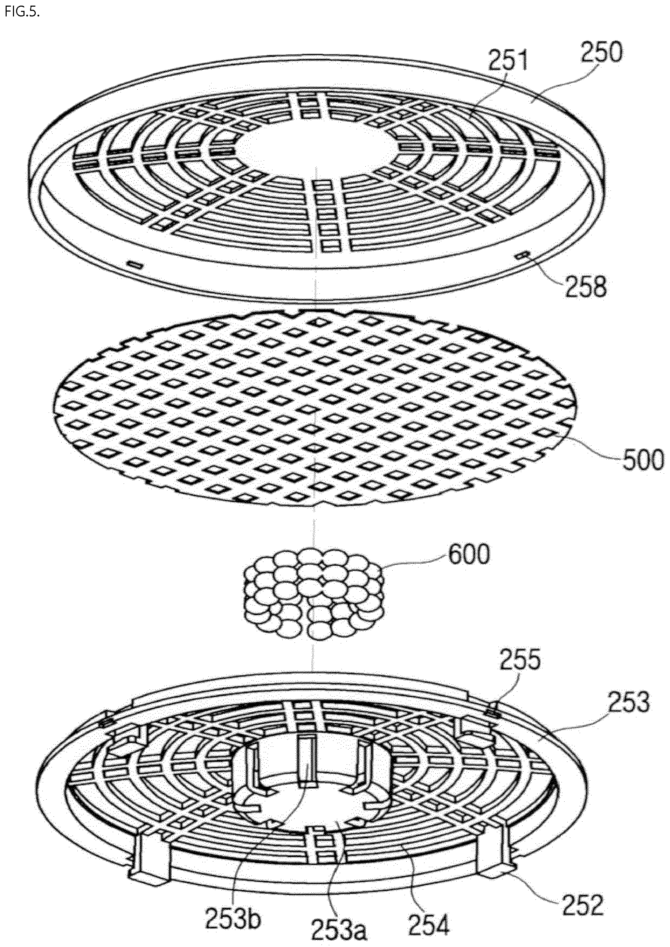

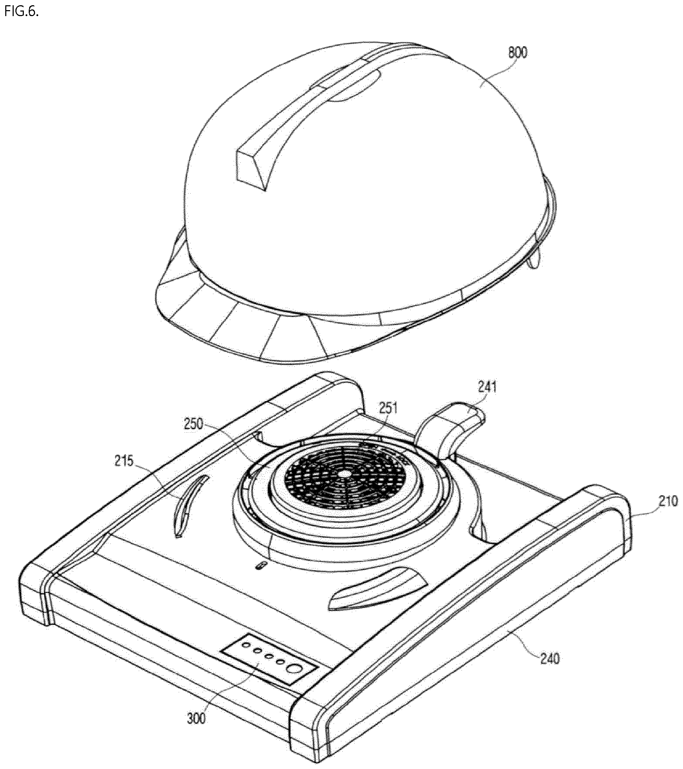

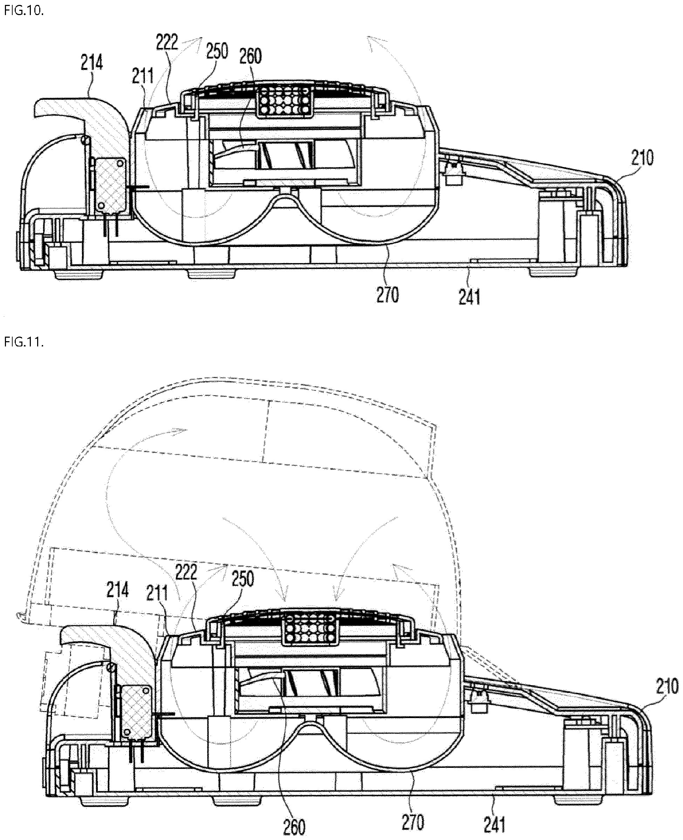

FIG. 3 is an exploded perspective view of a sterilizing/drying/deodorizing apparatus according to the present invention, FIG. 4 is an exploded perspective view of the sterilizing/drying/deodorizing apparatus according to the present invention, as seen from below, FIG. 5 is an exploded perspective view of a principal part extracted from the sterilizing/drying/deodorizing apparatus according to the present invention, FIG. 6 is a perspective view of the sterilizing/drying/deodorizing apparatus according to the present invention, FIG. 10 is a longitudinal-sectional view of the sterilizing/drying/deodorizing apparatus according to the present invention, and FIG. 11 is a longitudinal-sectional view illustrating the usage state of the sterilizing/drying/deodorizing apparatus according to the present invention.

A case main body 210 of the sterilizing/drying/deodorizing apparatus is formed to have a rectangular structure in which a right portion is slightly low and a left portion is slightly high in FIGs. 3 and 4. That is, since the sterilizing/drying/deodorizing apparatus is hung on the wall at an industrial site, the right portion of the case main body 210 corresponding to a lower part is configured to be slightly lower than the other portion of the case main body 210 so that a safety helmet is easily mounted on the case main body 210.

In addition, as shown in FIG. 3, a cylindrical installation hole part 211 which extends vertically is formed at the center of the upper portion of the case main body 210 to a designated height, and an air guide ring part 221 is formed at a predetermined interval from the inner circumferential surface of the installation hole part 211 so that an air vent 221 is provided between the installation hole part 211 and the air guide ring part 222. Here, the air vent 221 communicates with the inside of the case main body 210, as shown in FIGs. 10 and 11, and the upper ends of the installation hole part 211 and the air guide ring part 222 are inclined towards the central portion of the case main body 210 so that air exhausted through the air vent 221 is concentrated at the center of the safety helmet and is then absorbed into the case main body 210 by an intake fan 260, thereby minimizing leakage of air exhausted through the air vent 221 to the outside of the safety helmet and thus preventing pollution of an indoor space.

Further, a safety net 230 is formed on the upper portion of the inside of an opening of the air guide ring part 222, a installation protrusion part 219 having a plurality of fastening holes 220 is formed on the upper portion of the inside of the air guide ring part 222, and one side of each of the fastening holes 220 has a larger area and the other side of each of the fastening holes 220 extending from the one side has a slightly smaller area, as shown in FIG. 3.

Further, a safety helmet seated recess part 212 is formed in the upper portion of the case main body close to the installation hole part so that a safety band protruding from a hat, particularly, a safety helmet, may be stably mounted on the safety helmet seated recess part 212, as shown in FIG. 11, and a hanger part 214, on which the safety helmet is stably hung, is installed at the center portion of the safety helmet seated recess part 212 and is fixedly inserted into a fitting hole (not shown) formed through the central portion of the safety helmet seated recess part 212 to be opened upwards.

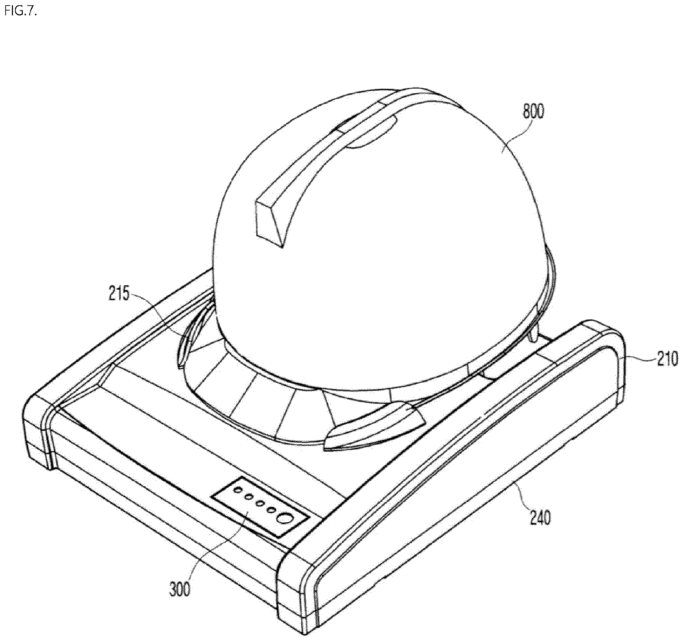

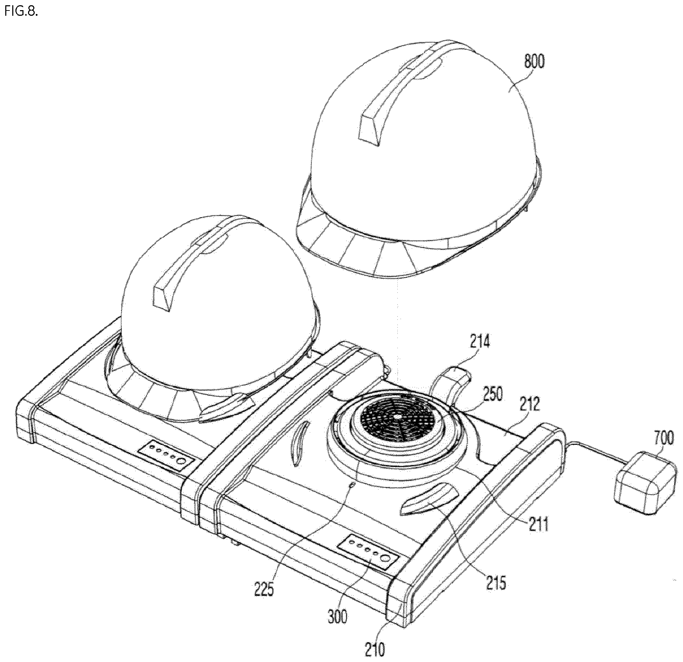

In addition, fixing holders 217, into which a front surface portion of the safety helmet forming a visor may be stably and fixedly inserted, are installed at both sides of the lower portion of the installation hole part 217, insertion grooves 215 having a curvature of a designated depth are formed in both sides of the lower portion of the installation hole part 211, screw parts are formed in the insertion grooves 215, and screw parts 218 extending downwards from the fixing holders 217 are engaged with the screw parts in the insertion grooves 215. Here, it is important to stably and fixedly insert side portions of the visor of the safety helmet into the inner surfaces of the fixing holders 217 installed at both sides of the case main body 210, as shown in FIGs. 7 and 8, and a worker may selectively hang a contaminated safety helmet on the hanger part 214 of the case main body 210 or fix the safety helmet to the fixing holders 217.

Finally, a plurality of fastening bosses 211a, 211b and 211c arranged at designated intervals is formed on the open bottom surface of the case main body 210.

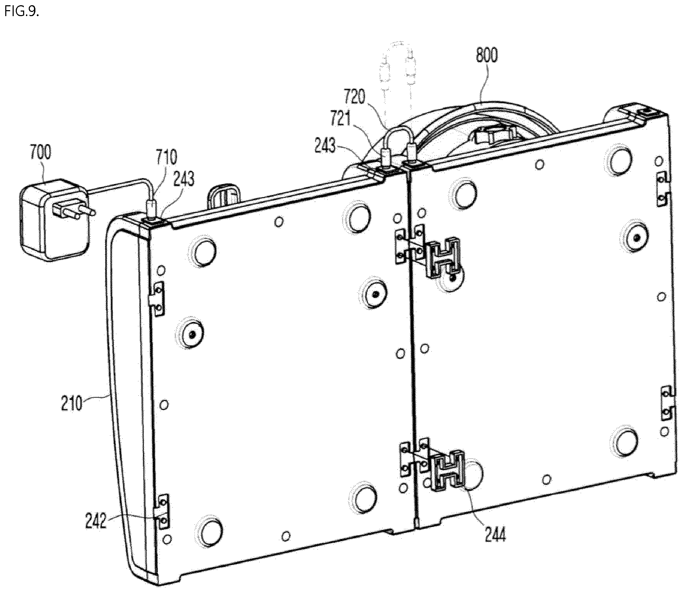

A case bottom plate 240 is installed under the open bottom surface of the case main body 210, and a plurality of fastening bosses 241 corresponding to the fastening bosses 211a, 211b and 211c of the case main body 210 is formed on the case bottom plate 240 and is firmly coupled to the fastening bosses 211a, 211b and 211c by screws, as shown in FIGs. 4 and 9.

Further, a plurality of case main bodies 210 is connected by forming connection grooves 242, which are opened sideways to a designated depth, at both side portions of the case bottom plates 240 of two case main bodies 210 which are arranged close to each other, inserting one H-shaped connection holder 244 into the neighboring connection grooves 242 of the case main bodies 210, which are close to each other, and then coupling the connection holder 244 to the connection grooves 242 using screws.

Further, a female terminal part 243 to which power may be applied is formed at each of both sides of the case bottom plate 240 of each case main body 210, a male terminal part 710 connected to an external power supply 700 is connected to one female terminal part 243 of the case bottom plate 240, which is located at the outermost position, and power is supplied to other case bottom plates disposed close to the case bottom plate 240 connected to the external power supply 700 using a jump wire 720 having male terminal parts 721 provided at both ends thereof.

Further, the intake fan 260 is installed close to the safety net 230 installed on the bottom surface of the center of the case main body 210, and is screwed to the fastening bosses 211b of the case main body 210.

In addition, an air induction part 270 corresponding to the installation hole part 211 protruding from the bottom surface of the center of the case main body 210 is installed, and the air induction part 270 has an air distribution protrusion 272 having a conical shape which protrudes upwards from the central portion of the air induction part 270, to uniformly distribute external air suctioned by the intake fan 260 without colliding therewith and simultaneously to raise the air along the inner circumferential surface of the air induction part 270 so as to exhaust the air to the air vent 221 between the installation hole part 211 and the air guide ring part 222.

A filter case 250 is detachably installed on the upper surface of the safety net 230 of the case main body 210, an installation net 251 is formed on the upper surface of the filter case 250, a disc-shaped filter member 500 is installed under the bottom surface of the installation net 251, a net-shaped fixing frame body 253 is coupled to the bottom surface of the filter member 500 so as to stably fix the filter member 500 from below, a deodorizing ball accommodation cabinet 253a having a plurality of intake holes 253b is formed on the central portion of the bottom surface of the fixing frame body 253, deodorizing balls 600, which may remove unpleasant smells from air primarily passed through the filter member 500, completely fill the inside of the deodorizing ball accommodation cabinet 253a through an open upper surface thereof, and the open upper surface of the deodorizing ball accommodation cabinet 253a is closed by the filter member 500, as shown in FIG. 5.

Further, fixing protrusions 255 are formed at the edge of the fixing frame body 253 and coupled to fixing holes 258 formed in the inner circumferential surface of the filter case 250, and thus, the fixing frame body 253 is not separated from the filter case 250 even when subjected to external shocks.

In addition, L-shaped fastening parts 252 corresponding to the fastening holes 220 formed in the upper portion of the inside of the air guide ring part 222 are formed on the bottom surface of the fixing frame body 253, one side surface of a bent end of each of the fastening parts 252 is thinner than the other side surface of the bent end so that the filter case 250 may be very easily rotated in one lateral direction in the state in which the fastening parts 252 are inserted into the fastening holes 220, and the bent ends of the fastening parts 252 closely contact the bottom surface of the installation protrusion part 219 so as to stably fix the filter case 250.

Ion generators 400 which may remove smells and various bacteria from inflowing air are respectively installed on both sides of the air induction part 270 within the filter case 250, as shown in FIG. 4.

Further, a control member 300 having a plurality of operation buttons so as to control operation of the intake fan 260 and the ion generators 400 for a designated time is installed within the filter case 250, and the operation buttons of the control member 300 are exposed from the upper surface of the case main body 250.

In addition, a sensor unit 800, such as an infrared proximity sensor, is installed within the case main body 250, and the sensor unit 800 is exposed upwards through a sensor hole 225 formed through the upper surface of the case main body 250, as shown in FIG. 3, and may thus operate the control member 300 for a designated time when the safety helmet is mounted on the case main body 250. Here, the sensor unit 800 may be installed at other positions, for example, at the safety helmet hanger part 240, and a switch unit which is operated by the weight of the safety helmet may be installed at the mounting part 240.

The functions and effects of the sterilizing/drying/deodorizing apparatus having the above configuration according to the present invention will be described in detail as follows.

First, as shown in FIGs. 3 to 5, the disc-shaped filter member 500 is installed under the bottom surface of the installation net 251 of the filter case 250 and simultaneously the net-shaped fixing frame body 253 is coupled to the bottom surface of the filter member 500 so as to stably fix the filter member 500 from below, the deodorizing balls 600, which may remove unpleasant smells from air primarily passed through the filter member 500, completely fill the inside of the deodorizing ball accommodation cabinet 253a, which is formed on the lower surface of the central portion of the fixing frame body 253, through the open upper surface thereof, and then, the open upper surface of the deodorizing ball accommodation cabinet 253a is closed by the filter member 500.

When the fixing frame body 253 is coupled to the filter case 250, the filter case 250 is coupled to the case main body 210, and in this case, the L-shaped fastening parts 252 formed on the bottom surface of the fixing frame body 253 are inserted into the fastening holes 220 formed in the upper portion of the inside of the air guide ring part 222 and then the filter case 250 is rotated in one lateral direction.

Here, when the filter case 250 is rotated in one lateral direction, the bent ends of the fastening parts 252 closely contact the bottom surface of the installation protrusion part 219 and thus the filter case 250 may be stably fixed.

When the filter case 250 is coupled to the case main body 210, the intake fan 260 is installed on the bottom surface of the center of the case main body 210, the air induction part 270 is installed so as to correspond to the installation hole part 211 protruding from bottom surface of the center of the case main body 210, and finally the case bottom plate 240 is coupled to the bottom surface of the case main body 210 using screws.

In order to connectively install a plurality of case main bodies 210, the case main bodies 210 are arranged such that the connection grooves 242 formed in both side portions of the case main bodies 240 are connected to each other, and then one H-shaped connection holder 244 is inserted into the connection grooves 242, which are connected, and is coupled to the connection grooves 242 using screws, thereby connecting the case main bodies 210, as shown in FIGs. 8 and 9./

Thereafter, the male terminal part 710 connected to the external power supply 700 is connected to one female terminal part 243 of the case bottom plate 240, located at the outermost position among the female terminal parts 243 provided at both sides of the connected case bottom plates 240, and the male terminal parts 721 of the jump wire 720 are connected in series to the corresponding female terminal parts 243 of two connected case bottom plates 240, and thereby, power is applied to all of the connected case bottom plates 240.

In order to wash a contaminated safety helmet using the above-described sterilizing/drying/deodorizing apparatus, the safety helmet to remove contaminants therefrom is hung on the hanger part 214 of the case main body 250, or a visor of the safety helmet is inserted into the fixing holders 217 of the case main body 250, and thereby, the visor is in surface contact with the curved inner surfaces of the fixing holders 217 and thus separation of the safety helmet from the apparatus may be prevented.

As described above, when the safety helmet is mounted on the case main body 250, the sensor unit 800 installed within the case main body 250 senses the safety helmet and thus operates the control member 300, and in this case, the ion generators 400 within the case main body 250 are operated to prepare for sterilization and deodorization of the safety helmet, and simultaneously, the intake fan 260 is operated to suction contaminants into the case main body 210 from the safety helmet.

That is, when the intake fan 260 is operated, as shown in FIGs. 10 and 11, the contaminants suctioned from the inside of the safety helmet pass through the filter member 500, and thus contaminants including fine dust and various bacteria are primarily removed.

Air having passed through the filter member 500 is suctioned into the deodorizing ball accommodation cabinet 253a formed at the central portion of the fixing frame body 253 installed under the bottom surface of the filter member 500, and is thus filtered so as to remove unpleasant smells through the deodorizing balls 600 accommodated in the deodorizing ball accommodation cabinet 253a, and the filtered air is suctioned into the air induction part 270 of the case main body 210 through the intake holes 253b formed through the deodorizing ball accommodation cabinet 253a.

The air introduced into the air induction part 270 is uniformly distributed by the air distribution protrusion 272 protruding upwards from the central portion of the air induction part 270, and is simultaneously raised along the inner circumferential surface of the air induction part 270 having a curved surface, thereby being exhausted to the air vent 221 between the installation hole part 211 and the air guide ring part 222 and then being injected to the inner surface of the safety helmet.

That is to say, as shown by arrows of FIG. 11, when fresh and clean purified air is exhausted to the edge of the inner surface of the safety helmet through the air vent 221, as the air is gathered in the center of the safety helmet, the air contains contaminants remaining in the safety helmet, and, here, suction of the contaminated air into the air induction part 270 by suction force of the intake fan 260 located at the center of the case main body through the above-described process is repeated for a designated time, thereby sterilizing, drying and deodorizing the safety helmet.

### (Description of Reference Numerals and Marks)

210: case main body 211: installation hole part
212: safety helmet seated recess part 214: hanger part
219: installation protrusion part 220: fastening hole
221: air vent 222: air guide ring part
240: case bottom plate 244: connection holder
243: female terminal part 253: fixing frame body
253a: deodorizing ball accommodation cabinet 253b: intake hole
260: intake fan 270: air induction part
272: air distribution protrusion 300: control member
400: ion generator 500: filter member
600: deodorizing ball 800: sensor unit

## Claims

1. A sterilizing/drying/deodorizing apparatus for hats including safety helmets, the sterilizing/drying/deodorizing apparatus comprising:
a case main body configured to have a cylindrical installation hole part formed at a center of an upper portion thereof so as to extend vertically, an air guide ring part formed at a predetermined interval from an inner circumferential surface of the installation hole part so that an air vent is provided between the installation hole part and the air guide ring part, a safety net formed on an upper portion of an inside of the air guide ring part, a installation protrusion part configured to have a plurality of fastening holes formed in the upper portion of the inside of the air guide ring part, a safety helmet seated recess part formed in an upper portion of the case main body close to the installation hole part so that a safety band protruding from a hat, including a safety helmet, is stably mounted on the safety helmet seated recess part, a hanger part installed at a center of the safety helmet seated recess part so that the safety helmet is stably hung on the hanger part, fixing holders installed at both sides of a lower portion of the installation hole part so that a front surface portion of the safety helmet is stably and fixedly inserted into the fixing holders, and a plurality of fastening bosses formed on an open bottom surface of the case main body;
a case bottom plate coupled to the open bottom surface of the case main body by a plurality of fastening bosses and screws;
an intake fan fixedly coupled to a bottom portion of the installation hole part of the case main body close to the safety net;
an air induction part connected to an end of the installation hole part of the case main body and configured to have an air distribution protrusion configured to have a conical shape and to protrude upwards from a central portion of the air induction part to suction external air by the intake fan without colliding therewith and to raise the air along an inner circumferential surface of the air induction part so as to exhaust the air to the air vent between the installation hole part and the air guide ring part;
a cylindrical filter case configured to have an installation net formed on an upper surface of the filter case, a disc-shaped filter member installed under a bottom surface of the installation net, a net-shaped fixing frame body coupled to a bottom surface of the filter member so as to stably fix the filter member from below, a deodorizing ball accommodation cabinet including a plurality of intake holes formed in a central portion of a bottom surface of the fixing frame body, and deodorizing balls configured to completely fill an inside of the deodorizing ball accommodation cabinet through an open upper surface thereof so as to remove unpleasant smells from air primarily passed through the filter member, wherein the open upper surface of the deodorizing ball accommodation cabinet is closed by the filter member, and fastening parts corresponding to the fastening holes of the installation protrusion part on the upper portion of the air guide ring part are formed on the bottom surface of the fixing frame body;
ion generators respectively installed on both sides of the air induction part to remove smells and various bacteria from inflowing air; and
a control member configured to have a plurality of operation buttons so as to control operation of the intake fan and the ion generators installed within the case main body for a designated time.

2. The sterilizing/drying/deodorizing apparatus according to claim 1, wherein a sensor unit is installed on an upper surface of the case main body so as to operate the control member for a designated time when the safety helmet is mounted on the case main body.

3. The sterilizing/drying/deodorizing apparatus according to claim 1, wherein two case main bodies arranged close to each other are stably fixed to each other by forming connection grooves configured to be opened sideways to a designated depth in both side portions of each of case bottom plates of the case main bodies and inserting one H-shaped connection holder into neighboring connection grooves of the case main bodies, which are close to each other.

4. The sterilizing/drying/deodorizing apparatus according to claim 1, wherein female terminal parts configured to receive applied power are respectively formed at both sides of each of case bottom plates of case main bodies, an external power supply is connected to one female terminal part of the case bottom plate, located at an outermost position, and power is supplied to other case bottom plates disposed close to the case bottom plate connected to the external power supply using a jump wire having male terminal parts provided at both ends thereof.
